# EUROPEAN PATENT SPECIFICATION

(11) **EP 2 911 730 B1**
(45) Date of publication and mention of the grant of the patent: **15.05.2019**
(21) Application number: 13849732.6
(22) Date of filing: 24.09.2013
(51) Int. Cl.: A61M 25/01, A61M 25/00

(54) **CATHETERS AND RELATED EQUIPMENT**
KATHETER UND ENTSPRECHENDE AUSRÜSTUNG
CATHÉTERS ET MATÉRIEL ASSOCIÉ

(30) Priority: 25.10.2012 US 201213660790
(43) Date of publication of application: 02.09.2015
(73) Proprietor: Bayer Healthcare LLC, Whippany, NJ 07981-1544 (US)
(72) Inventor: UBER, Arthur, E., Pittsburgh, PA 15208 (US); THOR, Eric, J., Arden Hills, MN 55112 (US); BONNETTE, Michael, J., Minneapolis, MN 55408 (US); GRIFFITHS, David, M., Pittsburgh, PA 15208 (US); SMITH, Kane, Pittsburgh, PA 15219 (US); MCDANIEL, Barry, Lynn, Pittsburgh, PA 15237 (US)
(74) Representative: Browne, Robin Forsythe
(86) International application number: PCT/US2013/061327
(87) International publication number: WO 2014/065969

(56) References cited:
- WO-A1-01/13789
- DE-A1- 2 817 974
- FR-A1- 2 552 332
- US-A- 3 703 174
- US-A- 3 703 174
- US-A- 3 826 256
- US-A- 4 243 033
- US-A- 4 243 033
- US-A- 5 772 636
- US-B2- 8 021 321

## Description

### CROSS-REFERENCE TO RELATED APPLICATIONS

This application claims the benefit of continuation-in-part U.S. application Ser. No. 13/660,790, filed on October 25, 2012, which is a continuation-in-part application of U.S. application Ser. No. 12/297,637 filed Oct. 17, 2008, which is a national phase application of International Application No. PCT/US07/66930 filed Apr. 19, 2007, and entitled "Central Venous Catheters and Related Equipment," which claims the benefit of U.S. Provisional Application No. 60/794,051, filed Apr. 21, 2006.

### BACKGROUND

Catheters have long been used for injecting fluids into the central venous circulation. Needles and relatively short peripherally inserted venous catheters (PIVC) are used for routine blood draws and fluid administrations. Central catheters discharge fluids centrally in the venous vasculature, and are commonly used for administering drugs that are too damaging to the veins to administer peripherally, for example, some chemotherapy, antibiotics, and parenteral nutrition. If the catheter and/or the veins are large enough and strong enough, they can be used for the rapid injection of contrast agents for imaging procedures such as, for example, CT, MR, ultrasound, and molecular imaging studies. PIVC lines are generally inexpensive and can be placed or installed by normal nurses or in some cases by specially trained phlebotomists.

Longer central catheters and infusion ports are, generally, placed into veins in the chest or neck and usually require surgery to be inserted into the vein. More recently, long flexible catheters generally referred to as peripherally inserted central catheters (PICC) have replaced infusion ports that are surgically implanted. These PICC lines can be inserted through a vein in the arm into the central venous circulation near the heart by trained nurses providing a more economic and patient friendly means for inserting a central catheter or infusion port. Generally, a guide wire is provided in the lumen of the flexible catheter to provide rigidity during the insertion procedure and a stiff needle, optionally with a dilator, is used to gain access to the vessel. The insertion procedure is carried out using a fluoroscope (or an ultrasonic imaging device) to help the user guide the catheter through the vessel into the central vena cava and to confirm proper placement of the catheter tip. Once in place, the needle or guide wire is removed, leaving the flexible catheter with the distal tip properly positioned for injection of fluid. These catheters can be left in place for days to months for the low flow-rate infusion of medication into the patient, and/or for sampling blood in patients with veins that have been compromised by disease or by the corrosive effects of chemotherapeutic drugs. The issues with PICC placement, phlebitis, and irritation or damage to the vessels and/or the heart have been made worse to some extent by the increased use of Power PICCs which can accommodate the pressures generated during the injection of CT contrast. This is because they often are made of a stiffer, stronger plastic and similarly may be larger in size to provide sufficient flow rates for the use in imaging procedures US 4,243, 033 discloses a catheter retention device including a catheter retention body defining a lumen designed to receive a catheter, the body having a proximal end and a distal end, wherein the proximal end has an aperture for entry of a catheter and the distal end has an aperture for exit of the catheter. Side ports are provides which allow for connection of a syringe for injection of a fluid into the catheter retention device.

Similar prior art catheter retention devices are known from documents FR 2,552,332, DE 2,817,974, US 3,826,256 and US 3,703,174.

However, insertion of a PICC line has several challenges and drawbacks. The long, relatively stiff catheter and/or guide wire requires the creation and maintenance of a large sterile field around the insertion point so as to not contaminate the catheter or guidewire before insertion into the body. During insertion, the PICC line can catch on valves and tight bends in blood vessels, potentially causing trauma to blood vessels. In addition, because of the sometimes tortuous path of the veins, it can be difficult to move or remove the guidewire relative to the catheter during installation or when installation is complete. Similarly, a stiff tip on the PICC line can irritate a patient's heart if the PICC line is inserted too far or damage the superior vena cava if not inserted far enough. If the catheter is too large or stiff, it can damage the peripheral vein through which passes. This can lead to complications such as thrombosis, pain, and infection. Because of the importance of the correct placement of the catheter tip, the procedure was historically done under fluoroscopy in an interventional suite. At the location where the catheter exits the patient's skin, the stiffness of the catheter also increases the likelihood of motion and disruption of the seal between the catheter and the skin which can increase the possibility of infection.

### SUMMARY OF THE INVENTION

The embodiments described herein provide one or more peripherally inserted central access catheters with one or more lumens which minimize trauma to the vessels. The controlled delivery of fluid may be used to help urge the catheter distally and/or to dilate the vein for easier insertion. Optionally it provides one or more proximally discharged lumens which optionally have the capability for accommodating the power injection for venous drug injections commonly used as part of an imaging procedure. Optionally the central access catheter can be inserted through an existing peripheral access device which can remain in place or be removed.

References to an "embodiment" or "embodiments" or "inventions" throughout the description which are not under the scope of the appended claims merely represent possible exemplary executions and are therefore not part of the present invention.

Some embodiments are directed to a catheter retention device that includes a catheter retention body having a proximal and a distal end, the catheter retention body defining a lumen designed and configured to receive a catheter; at least one distal lateral fitting on a distal portion of the catheter retention body, the lateral fitting being designed and configured to couple to a fluid source and introduce fluid into the distal portion of the lumen of the catheter retention body; and a distal fitting at a distal end of the catheter retention body, the distal fitting defining an aperture through which fluid and a catheter exit the lumen of the catheter retention body. In such embodiments, fluid flowing through the aperture may provide substantially all of the force required to expel the catheter from the catheter retention body.

Other embodiments are directed to a multi-port introducer including an introducer tube, designed and configured to be inserted into a blood vessel, the introducer tube having an opening with a diameter sufficient to accommodate a catheter and allow fluid delivery through the introducer tube during deployment of the catheter; a multi-port fitting head operably connected to the introducer tube; a first fitting operably connected to the multi-port introducer, the first fitting configured to provide a substantially straight path from a proximal opening of the first fitting to the opening in the distal end of the introducer tube; and one or more second fittings operably connected to a lateral portion of the multi-port introducer.

Still other embodiments are directed to methods for introducing a catheter into a blood vessel, the methods include the steps of introducing an angiocatheter into a blood vessel; and simultaneously introducing fluid and a catheter into the blood vessel.

### BRIEF DESCRIPTION OF THE DRAWINGS

In the following detailed description, reference is made to the accompanying drawings, which form a part hereof. In the drawings, similar symbols typically identify similar components unless context dictates otherwise. The illustrative embodiments described in the detailed description, drawings, and claims are not meant to be limiting. Other embodiments may be utilized and other changes may be made, without departing from the scope of the subject matter presented herein. It will be readily understood that the aspects of the present disclosure, as generally described herein and illustrated in the Figures, can be arranged, substituted, combined, separated, and designed in a wide variety of different configurations, all of which are explicitly contemplated herein.
**FIG. 1** is an illustration of an angiocatheter.
**FIG. 2A** is an illustration of a catheter retention device.
**FIG. 2B** is an illustration of a catheter retention device attached to an angiocatheter.
**FIG. 3A** is an illustration of a catheter retention device showing the location of exemplary fittings.
**FIG. 3B** is an illustration of a catheter retention device showing the location of exemplary fittings and valves.
**FIG. 4A** is an illustration showing a catheter retention device having a catheter wound within the lumen.
**FIG. 4B** is an illustration showing a catheter retention device having a catheter folded within the lumen.
**FIG. 4C** is an illustration showing a catheter retention device having a catheter wound in a spherical catheter retention device.
**FIG. 4D** is an illustration showing a catheter retention device having a catheter wound in a spherical catheter.
**FIG. 5** is an illustration showing an exemplary catheter.
**FIG. 5A** is an illustration of a cross-section of a single lumen catheter.
**FIG. 5B** is an illustration of a cross-section of a double lumen catheter.
**FIG. 6** is an illustration of a catheter showing the locations of exemplary lateral slits.
**FIG. 7** is an illustration of a multi-port introducer and a needle deployment device.
**FIG. 8** is an illustration of a multi-port introducer connected to a catheter retention device.
**FIG. 9** is a schematic diagram showing an exemplary control system for deploying a catheter.

### DETAILED DESCRIPTION

As used in this document, the singular forms "a," "an," and "the" include plural references unless the context clearly dictates otherwise. Unless defined otherwise, all technical and scientific terms used herein have the same meanings as commonly understood by one of ordinary skill in the art. Nothing in this document is to be construed as an admission that the embodiments described in this document are not entitled to antedate such disclosure by virtue of prior invention.

This disclosure is not limited to the particular systems, devices and methods described, as these may vary. The terminology used in the description is for the purpose of describing the particular versions or embodiments only, and is not intended to limit the scope.

The word "proximal" refers to a direction relatively closer to a clinician using the device described herein, and the word "distal" refers to a direction relatively further from the clinician. For example, the end of a catheter placed within the body of a patient is considered a distal end of the catheter, while the catheter end remaining outside the body is a proximal end of the catheter.

Embodiments of the invention include devices useful for inserting a catheter into a blood vessel that provide for flow of fluid into the blood vessel simultaneously with insertion of the catheter. Simultaneous fluid flow with the insertion of a catheter allows the catheter to be carried into the blood vessel with the flow of fluid without need for a significant force from the back or distal end pushing the catheter into the blood vessel. In addition, the influx of fluid during insertion increases the blood volume in the blood vessel, expanding and opening the vein to facilitate insertion and centrally directed movement, and the flow rate of the blood through the vessel thereby providing sufficient blood flow to carry the catheter through the circulatory system without the need to provide a proximally directed force by distally pushing the catheter into the blood vessel. Once in the vessel, the catheter proceeds through the blood vessel at about the same rate as the flow rate of the blood or slower, so that there is a proximally directed force at the tip and optionally over much of its length caused by drag as the fluid moves past the catheter, reducing the likelihood of kinking that can happen when the catheter exceeds the velocity of the blood and the body of the catheter pushes past the tip during insertion.

The need for guidewires and other stiffening means associated with the catheter during insertion is also reduced or eliminated as well as allowing catheters to be prepared from softer or more flexible materials such as silicones or softer polyurethanes, or have smaller outside diameter and thinner walls than is possible using catheters and methods of the prior art. Reducing or eliminating the need for guidewires also allows for the use of coated or multi-layer catheter tubes. For example, in certain embodiments, the catheter may be coated or include a layer of hydrophobic drugs on the inner surface of the catheter while the outer surface can be chosen from materials that are compatible with the blood or that include, for example, a coating of anti-coagulant or anti-fibrotic drug. Eliminating guidewires also allows for variation in the diameter of the inner and outer diameter and variation in the material stiffness over the length of the catheter. For example, in some embodiments, the diameter of the catheter may be decreased relative to the body of the catheter to reduce the likelihood of trauma caused by the tip or reduce the total volume of fluid in the lumen.

Additional embodiments are directed to catheters having a variety of new features, catheter retaining devices that allow coiled catheters to be contained within a shortened sterile housing before, during, and after insertion of the catheter into the blood vessel, and methods for using the insertion devices, catheters, and catheter retaining devices. Various such embodiments may allow for a reduced sterile field size, enable accurate tip placement by making it easier to adjust the location of the catheter tip in both upstream and downstream directions, and reduce the need for cutting of the catheter to the appropriate length before insertion into the vein.

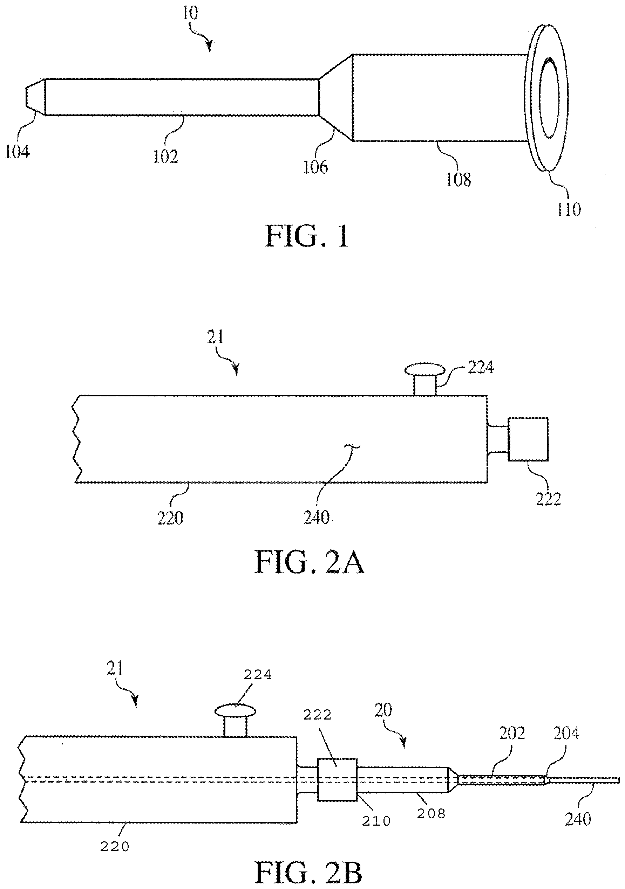

The devices of various embodiments include a means for introducing fluid into the blood vessel while simultaneously inserting a catheter into a blood vessel. Means for introducing fluid into a blood vessel are well known and used in the art. For example, short peripherally inserted catheters, angiocatheters, are commonly used to introduce intravenous (IV) fluids into a blood vessel. As depicted in FIG. **1****,** such angiocatheters **10** generally include a short insertion tube **102** having an angled, conical, or tapered distal end **104,** and a Venturi feature or other tapered portion **106** that connects the short insertion tube **102** to a body **108.** The body **108** generally includes a proximal fitting **110** that allows the angiocatheter **10** to be connected to tubing associated with fluid source such as an IV bag or syringe for infusion of a drug or other agent. A needle, not shown, extends through the lumen of tube **102** and is use to puncture the skin, tissue and blood vessel wall. Once in the vessel, the tube **102** is pushed over the needle into the vessel and the needle is withdrawn and discarded.

Certain embodiments of the invention include catheter retaining devices that are configured to attach to the fitting of angiocatheters or short peripherally inserted venous catheters (PIVC) like the angiocatheter **10** illustrated in FIG. **1****.** The ability to deliver a longer catheter through an existing angiocatheter without having to make a new IV stick may provide a benefit in some emergency and critical care situations, or in patients where finding a vein is difficult.

As illustrated in FIG. **2A** such catheter retaining devices **21** may include a catheter retention body **220** having a lumen sized and shaped to hold a central catheter **240,** a distal fitting **222** configured to attach to the fitting of an angiocatheter, and one or more lateral fittings **224** configured to attach to a fluid source. In some embodiments, the lateral fitting **224** may be at a distal portion of the catheter retention body **220.** FIG. **2B** shows the catheter retaining device **21** attached to a common angiocatheter **20.** In operation, fluid may enter the catheter retention body **220** through the lateral fitting **224.** The air in the catheter retaining body **220** can exit through distal fitting **222** prior to being connected to the angiocatheter **20.** After air has been removed and fluid has accumulated in the catheter retention body **220,** it can be connected to angiocatheter **20,** and the fluid will flow through the distal fitting **222** into the angiocatheter **20** through the proximal fitting **210** and the angiocatheter body **208.** The fluid will exit the angiocatheter **20** and enter the blood vessel through the distal end **204** of the insertion tube **202.** The flow of fluid through the devices will provide a pulling force on the catheter **240** related to the velocity or flow rate of the fluid flow. As the rate increases, the force will be sufficient to overcome the static friction holding the catheter in place and begin drawing the catheter from the catheter retention body **220** and into the blood vessel where blood flow enhanced by the additional fluid from the device will carry the catheter through the circulatory system to the site of deployment.

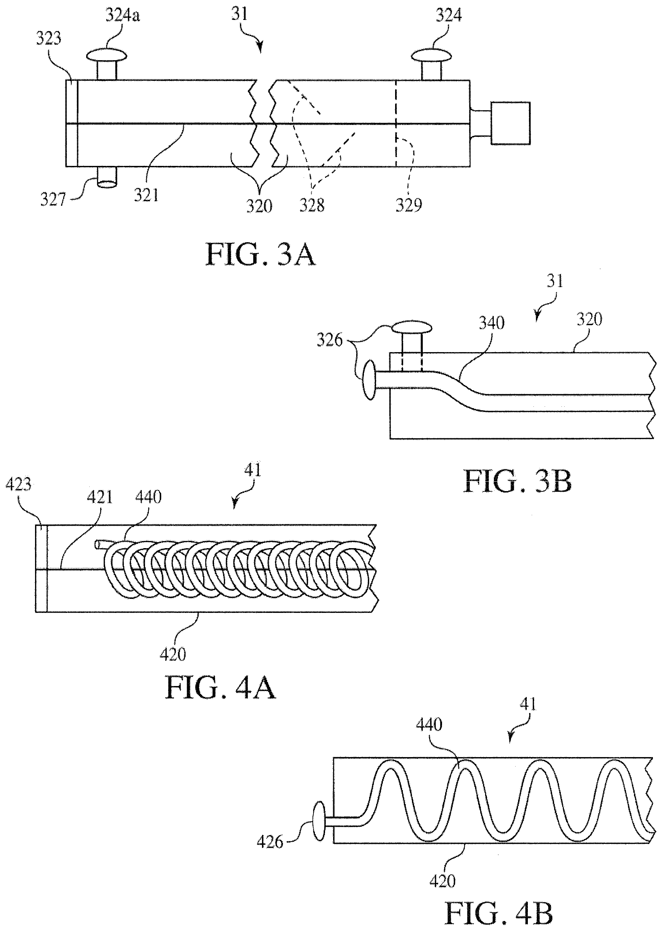

The catheter retaining devices of various embodiments may include any number of additional features necessary to control flow of fluid through the device, remove unwanted air or fluid from the central lumen of the catheter retention body **220,** control deployment rate or velocity of the catheter **240,** and direct the catheter through the catheter retaining device. For example as illustrated in FIG. **3A****,** in some embodiments, the catheter retaining device **31** may include one or more lateral fittings in addition to the distal lateral fitting **324** located along the catheter retention body **320,** and in certain embodiments, a second lateral fitting **324***a* may be positioned on a proximal portion of the catheter retention body. In such embodiments, the second lateral fitting **324***a* may provide an additional means for introducing fluid into the catheter retention body **320.** Alternatively or in addition, the second lateral fitting **324***a* may be on a distal portion of the catheter retention body **320** or the angiocatheter body so that fluid flowing through it exerts little or no force on the catheter while in the catheter retention body **320** or the angiocatheter body and flows into the vessel to distend and/or augment the flow in the vessel. The second lateral fitting may further provide a means for allowing air or fluid from within the catheter retention body **320** to leave the lumen as fluid enters through the distal lateral fitting **324.** In some embodiments, the catheter retaining device **31** may further include one or more one-way purge vents **327** that is capable of allowing air and fluid to exit the catheter retention body **320.**

The proximal portion of the catheter retention body may further include one or more fittings associated with the catheter. For example, as illustrated in FIG. **3B****,** in some embodiments, the catheter **340** may terminate at a catheter fitting **326** provided at a distal portion of the catheter retention body **320.** The catheter fitting **326** may provide a means for introducing fluids into the catheter **340** for delivery to the patient. As the catheters **340** of various embodiments may have more than one lumen, a separate catheter fitting may be provided for each lumen of the catheter **340.** Thus, the catheter retaining device **320** of embodiments may have one, two, three, or more catheter fittings **326** disposed on a distal portion of the catheter retention body **320.**

In some embodiments, the catheter retaining device **31** may further include one or more valves positioned to regulate the flow of fluid into and out of the catheter retention body **320.** For example, in some embodiments, a valve may be positioned at the distal lateral fitting **324,** and in other embodiments, a valve may be positioned at the catheter fitting **326.** In still other embodiments, the catheter retaining device **31** may include a valve at both the distal lateral fitting **324** and the catheter fitting **326.** In still other embodiments, the catheter retaining device **31** may include additional valves located anywhere along the catheter retention body, **320.** For example, valves may be provided with additional lateral fittings or valves associated with catheter fittings. The valves of various embodiments may be any type of valve known in the art including, for example, gate or slide valves, needle valves, check valves, diaphragm valves, butterfly valves, and like. These valves can be manually controlled or automatically controlled by the injection system to optimize the flow of fluid to and through catheter retention body **320** as well as the blood vessel to provide deployment force to the catheter as the catheter proceeds into veins as well as to ease movement to the central circulation.

In certain embodiments, the catheter retention body **320** may include texturing or features on an inner surface. Examples of such texturing include helical, longitudinal, circumferential grooves, rifling, projections, or other texturing. Texturing may allow a portion of an outer surface of the catheter to contact a portion of an inner surface of the catheter retention body **320** to maintain the position of the catheter in the lumen of the catheter retention body **320** and to provide friction to hold the catheter in place while allowing movement when acted on with sufficient force. In some embodiments, texturing or features may be provided to regulate and direct the flow of fluid through the catheter retention body **320** when the catheter is placed within the lumen of the catheter retention body **320.** For example, rifling of the inner diameter of the catheter retention body **320** may allow for directed flow of fluid within the second catheter around the catheter.

In particular embodiments, the catheter retention body **320** may include internal features such as, for example, one or more bulkheads **329** or baffles **328** positioned at intervals that segment portions of the catheter retention body **320** or provide currents that can provide additional pulling force on the catheter. In other embodiments, the baffles or bulkheads may be positioned to aid in the movement of the catheter through the catheter retention body by, for example, ensuring that the catheter is centered within the catheter retention body as it moves through the catheter retention body. In still other embodiments, the baffles or bulkheads may be positioned to aid in packaging of the catheter in the catheter retention body.

In certain embodiments, the catheter retaining device **31** may include a centering mandrel **321** sized to be received within a lumen of the catheter and a mandrel clamp **323** positioned to hold the centering mandrel at the center of a circumference of the catheter retention body **320.** In some embodiments, the centering mandrel **321** may be movably received by the mandrel clamp **323** such that the centering mandrel **321** may enter the catheter retention body **320** and be retained therein as the catheter progresses through the blood vessel.

There are several potential benefits to the inclusion of a centering mandrel **321.** It allows for a reduced flow rate of fluid necessary to introduce the catheter into the blood vessel by reducing the friction of the catheter on the catheter retention body **320** inner wall. The centering mandrel **321** positions the catheter such that an approximately equal amount of fluid flows on either side of the catheter, thereby optimizing the amount of insertion force exerted on the catheter by the fluid. The centering mandrel may also reduce the tendency of the catheter to bend or kink. A similar effect may be obtained by providing additional fluid deployment ports around the circumference of the deployment tube. Thus, lowered required flow rates may allow for hand-operated syringes to provide adequate fluid flow to insert the catheter into a patient. The centering mandrel may also provide some controlled friction to limit the rate of deployment by sizing of the fit or geometry. To fit within the catheter, the centering mandrel **321** may be smaller in diameter than the inner diameter of the catheter. For example, when used with a second catheter having an inner diameter of about 0.008 inches to about 0.035 inches or about 0.01. In this example, the centering mandrel **321** may have a diameter of about 0.008 inches or less.

As illustrated in FIG. **4A****,** the catheter retention body **420** may be designed to receive and package the catheter in any way. For example, in some embodiments, the centering mandrel **421** may be fixedly received by the mandrel clamp **423,** and the catheter **440** may be wound around the centering mandrel **421.** When wound around the centering mandrel **421,** the catheter may be in a single, double coiled, or triple coiled configuration. The centering mandrel **421,** in such embodiments, may provide for improved packaging of the catheter **440** that may allow for the size of the catheter retention body **420** to be reduced thereby reducing the overall size of the catheter retention device **41.**

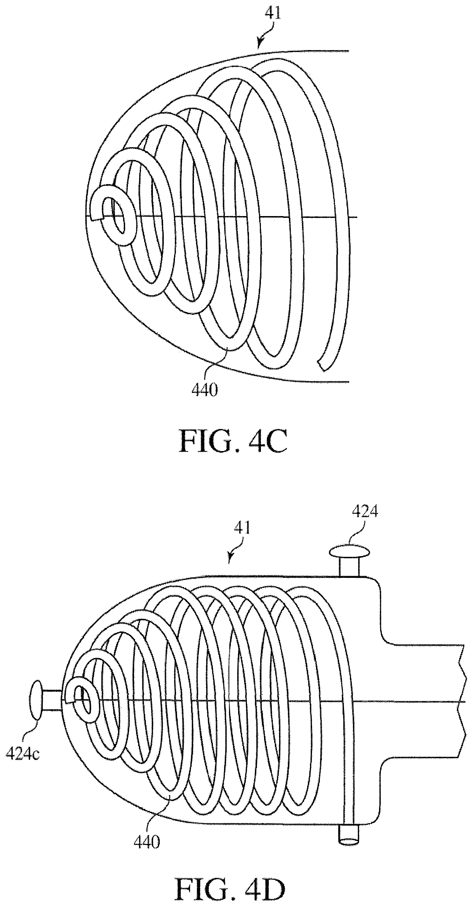

While the fixed centering mandrel **421** provides a means for winding or coiling the catheter 440 in the catheter retention body **420,** in some embodiments, the catheter may be wound or coiled in the catheter retention body **420** without a centering mandrel. In other embodiments, the catheter may be folded in the catheter retention body **420** as illustrated in FIG. **4B****.** The shape of the catheter retention body **420** may reflect the means by which the catheter is stored within the catheter retention body **420.** For example, in embodiments in which the catheter is coiled or wound, the catheter retention body **420** may have a substantially cylindrical, conical, or spherical shape, and in embodiments in which the catheter is folded, the catheter retention body **420** may have a square or rectangular box shape. Coiling the catheter on the inner surface of the catheter retention body **420** and pulling it off the inner surface may provide a compact, low friction deployment mechanism. In other embodiments, as illustrated in FIG. **4C** and FIG. **4D****,** the catheter **440** may be coiled upon itself, similar to a ball of twine that can be unwound from the inside out and the catheter retention device **41** may have a substantially spherical FIG. **4C** or oblong FIG. **4D** shape.

Because the catheter **440** can be wound, coiled, or folded within the catheter retention body **420,** the size of the catheter retention body may be compacted, i.e., shortened in length and larger in diameter thereby improving the hand ability of the catheter and catheter retention body. For example, in embodiments in which the catheter is not wound or coiled, the catheter retention body may have a length substantially equal to the length of the catheter, for example, from about 20 cm to about 70 cm or about 25 cm to about 65 cm and a diameter sufficient to receive a 4 French, 5 French, 6 French, or 7 French catheter. For example, the catheter retention body may have a lumen having an internal diameter of from about 1.5 mm to about 5 mm or about 2 mm to about 4 mm. In embodiments in which the catheter is wound or coiled within the catheter retention body, the length of the catheter retention body **420** may be substantially shortened while the diameter of the catheter retention body **420** may be increased. For example, length L of a cylindrical catheter retention body **420** may be about 5 cm to about 50 cm for a 4 French, 5 French, 6 French, or 7 French catheter having a length of about 20 cm to about 70 cm, and in other embodiments, the cylindrical catheter retention body **420** may have a length of about 10 cm to about 40 cm, about 15 cm to about 35 cm, or about 20 cm to about 30 cm, or any individual value within these exemplary ranges. Such cylindrical catheter retention bodies **420** may have an internal diameter D of about 10 mm to about 20 cm, about 50 mm to about 10 cm, or any individual value within these exemplary ranges. Similar sizes can be achieved for catheter retention bodies **420** that have square or rectangular box shapes. For example the length of a rectangular box shaped catheter retention body may be about 10 cm to about 40 cm, about 15 cm to about 35 cm, or about 20 cm to about 30 cm, or any individual value within these exemplary ranges, and the rectangular box shaped catheter retention body may have a height and width, which can be equal or different, of about 10 mm to about 20 cm, about 50 mm to about 10 cm, or any individual value within these exemplary ranges.

In some embodiments, the catheter may be introduced into the blood vessel using only the flow of fluid through the catheter retention device 41 and/or the introducer. In other embodiments, the catheter retention device may further include a means for manually or automatically reeling the catheter out of the catheter retention body and/or reeling the catheter back into the catheter retention body as may be useful during the procedure to provide for optimal final tip placement. For example, a handle may be disposed on an outer surface of the catheter retention body that may facilitate winding or unwinding of the catheter around a spindle inside of the catheter retention body **420,** and in other embodiments, a motor or motorized wheel, which can be operated manually or by an injection controller, may facilitate winding or unwinding of the catheter within the catheter retention body **420.** The means for reeling in and reeling out the catheter may allow the deployed catheter to be retracted, repositioned, or replaced without sacrificing sterility because the catheter will remain enclosed within the catheter retention body throughout the procedure. In other embodiments, the catheter retention body **420** may include one or more friction augmentation devices such as, for example, a pinch valve, iris, or other compression device that can be pulsed to control the feed-out of the catheter during insertion. Such friction augmentation devices can be controlled manually or by a system controller.

The catheters **440** contained within the catheter retention body **420** may be any type of catheter known in the art. For example, the catheters may include one or more lumens and can be configured as 4 French, 5 French, 6 French, or 7 French catheters. In certain embodiments, flow insertion and flow augmentation may allow for catheters having a small diameter, 1 French, 2 French, 3 French or as small as PE-10 tubing to be inserted. Such catheters can be configured to deploy any materials known in the art such as, for example, saline, active agents, drugs, medication, nutrients, or other foodstuffs. In some embodiments, the catheter may include a guidewire or other stiffening means that can be used during deployment of the catheter and then removed during use. In particular embodiments, catheters contained within the catheter retention body may not need to include a guidewire or stiffening means. Without wishing to be bound by theory, the increased blood volume created by the simultaneous administration of fluid during introduction of the catheter **440** may allow the catheter to be deployed without pushing the catheter into the blood vessel from its proximal end. Alternatively, the amount or amplitude of the primal pushing force can be reduced, while allowing for manual control by the nurse, providing a "power assist" for the catheter insertion procedure. The catheter is carried to the deployment site by the flow of blood which can be augmented by the flow of fluid and is less likely to kink as a result of portions of the catheter being pushed past the distal end of the catheter. As such, the need for stiffening means is reduced or eliminated, and moreover, in some embodiments, softer and more compliant materials may be used to make the catheter. In addition the vessels are dilated or enlarged by the augmented flow, thereby making insertion easier, less traumatic, and less problematic. Taken together, these improvements provide a catheter introduction system that is less likely to cause injury to the blood vessel.

As illustrated and discussed for the embodiment shown in FIG. **3B****,** the catheter **440** may terminate at a catheter fitting **426** provided at a distal portion of the catheter retention body **420.** The catheter fitting **426** may provide a means for introducing fluids into the catheter **440** for delivery to the patient. Further, placement of the distal lateral fitting **424** and the second lateral fitting **424***c* may vary with the various shapes of the catheter retention body **420.**

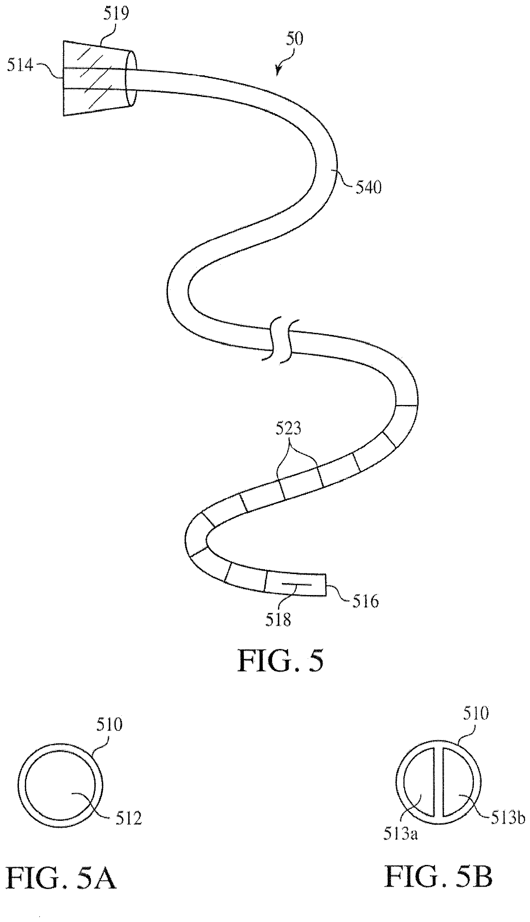

As illustrated in FIG. **5****,** the catheter **540** incorporated into the catheter retention devices described above may include features to facilitate insertion and/or facilitate interactions with the catheter retention device. Generally, the catheter **540** may have a distal end **516** configured to be inserted into a blood vessel, and a proximal end **514** that may include at least one opening (not shown) capable of allowing fluid to flow into and through the catheter **540** into the patient's vein. The proximal end **514** of the catheter **540** may be designed and configured to interact with an inner surface of the catheter retention body. For example, in some embodiments, a bulge or stopper **519** fixedly attached to the catheter may be provided at the proximal end of the catheter **540.** The bulge or stopper **519** may have any shape. For example, in some embodiments, the bulge or stopper **519** may be round or cylindrical, and in certain embodiments, the bulge or stopper **519** may have a cone shape. The bulge or stopper **519** may be sized to stop the second catheter **540** at an appropriate position within the catheter retention body to ensure that the catheter does not flow past the deployment site or completely enter the patient's blood vessel. Thus, in some embodiments, the bulge or stopper **519** may be sized to approximately equal the inner diameter of the catheter retention body and have a larger diameter than the distal fitting of the catheter retention body such that forward movement of the catheter **540** is stopped when distal fitting is reached.

In some embodiments, the catheter **50** may include marking features **523** spaced along the length of the catheter. Such marking features may show the length of the catheter and can be placed at equally spaced intervals such as, for example, each centimeter, each inch, or any suitable unit of measure known in the art. The markings **523** may provide a means for quickly customizing the length of the catheter **50** by cutting the catheter before it is introduced into the patient. In certain embodiments, the length of the catheter **50** may be determined before the catheter is packaged in, for example, a catheter retention body, to avoid cutting of the catheter during the introduction procedure. Because the catheter retention body is sterile, the sterility of the catheter is maintained while the catheter is in the catheter retention body, avoiding cutting eliminates the possibility of contamination during the introduction procedure. Alternatively, catheter may have a standard length and extra catheter can be pulled back and stored in the catheter retention body providing another means for avoiding cutting during the introduction procedure and potentially contaminating the catheter before introduction. Having markings extending towards or to the proximal end of the catheter can help in ascertaining that the correct length of catheter has been inserted into the patient.

Some embodiments of the invention are directed to catheters. Such catheters **50** generally include elongated, unitary, tubular structure made of a flexible, biocompatible material, such as silicone rubber that include a cylindrical wall **510** defining a longitudinal bore or lumen **512,** as illustrated in FIG. 5A. In some embodiments, the catheter **50** may include two or more longitudinal bores to provide multiple lumen catheters, and in particular embodiments the catheter may include two adjacent bores, which may have a substantially "D" shape **513***a*, **513***b*, in FIG. 5B. The catheters **50** of various embodiments may be sized to have a conventional diameter for catheters designed for insertion into blood vessels. For example, the catheters of embodiments may be 1 French, 2 French, 3 French, 4 French, 5 French, 6 French, or 7 French sized catheters having external diameters of about 0.6 mm to about 2.5 mm, or very thin tubing, such as PE10. The diameter of the lumen may vary based on the thickness of the sidewalls and the number of lumen associated with the catheter.

In some embodiments, the one or more lumens may extend throughout the length of the catheter **50,** and the catheter **50** may have at least one proximal opening **514** and at least one distal opening **516** through which fluids can flow into and out of the catheter **50.** In other embodiments, the one or more lumens may terminate before the end of the catheter. For example, in some embodiments, the catheter may have a proximal opening **514** and a sealed or otherwise closed distal end **516,** and the lumen may open to the blood vessel by one or more lateral slits **518** in the cylindrical wall **510** of the catheter **50** to provide a lateral opening. Such slits **518** may allow the opening to be created when fluid is introduced into the lumen **512, 513***a*, or **513***b*, and fluid pressure within the lumen may force the lateral slit **518** to open allowing fluid to exit the catheter. When the flow of fluid is stopped and fluid pressure within the lumen is decreased, the slit **518** may close keeping blood or other fluids from entering the catheter when fluid is not being introduced into the catheter **50.** Similarly, applying suction to the catheter will open the slit and allow blood to be drawn into the catheter for sampling as needed. A nonlimiting example embodiment is a Groshong catheter.

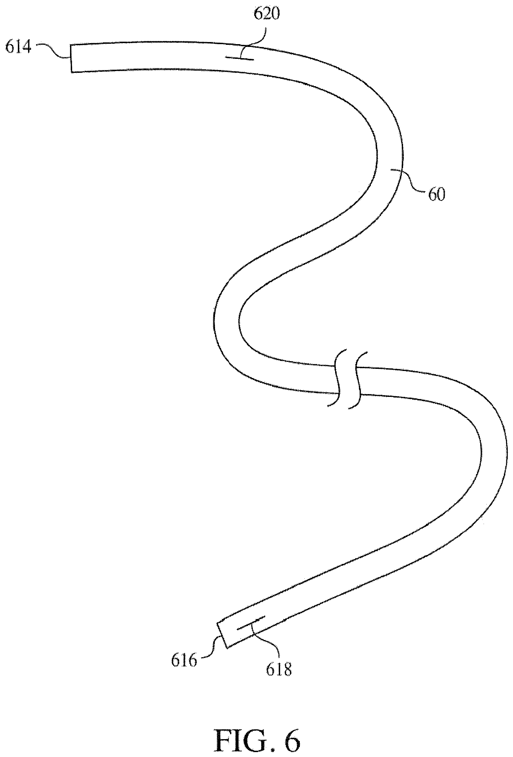

In certain embodiments, a multi-lumen catheter **50** such as the catheter illustrated in FIG. 5B may include more than one lateral slit. For example, as illustrated in FIG. 6, catheter **60** may include a distal lateral slit **618** at or near a distal end of the catheter **60,** and one or more additional lateral slits **620.** In some embodiments, the additional lateral slit **620** may be positioned at or near a proximal end **614** of the catheter **60.** In other embodiments, additional lateral slits **620** may be positioned at one or more positions throughout the length of the catheter **60.** In particular embodiments, the catheter **60** of such embodiments may be a multi-lumen catheter having at least two lumens such as the exemplary catheter illustrated in FIG. 5B. The distal lateral slit **618** may be associated with a first lumen **513***a*, and the one or more additional lateral slits **620** may be associated with a second lumen **513***b*. Thus, for example, the first lumen **513***a* may be configured to transport an active agent to the distal end **616** of catheter **60,** while the second lumen **513***b* is configured to transport a second active agent for more proximal administration. In some embodiments, the second lumen **513***b* having a proximal additional lateral slit **620** may be configured to transport medical fluid or saline that can be introduced into the blood vessel during introduction of the catheter **60** into the blood vessel, and the additional saline may act to speed blood flow through the blood vessel and aid in the movement of the catheter **60** through the blood vessel. In embodiments in which the catheter **60** includes two or more additional lateral slits **620** configured to transport medical fluid, medical fluid may be released over the entire length of the catheter during introduction of the catheter **60** into the blood vessel. The combined local effects of each additional lateral slit may allow for improved blood flow by increasing blood volume from the site of insertion of the catheter **60** to the final deployment site. Holes or slits positioned along the body of the catheter **60** or at a distal end **616** of the catheter **60** may allow fluid flow within the vessel to expand the vessel and ease insertion of the catheter **60** during insertion. After introduction of the catheter **60,** medical fluid may continue to be administered through the additional lateral slits **620,** or fluid flow may be stopped, and in some embodiments, active agents, drugs, medication, nutrients, or other foodstuffs may be administered through the patent through the second lumen **513***b* and additional lateral slits **620.** Proximal slit or opening to deliver CT contrast and prevent rupture if softer materials or smaller diameters are used. In some embodiments, the separation of the proximal and the distal ports may be sufficient to provide central vascular access for slow or moderate flows through a more atraumatic catheter section or segment and enable proximal access in a more peripheral vessel for higher or power injected flows.

In some embodiments, the catheter **60** may have a single lumen that has one or more distal slits or openings for distal delivery and/or withdrawal as well as normally closed proximal slits or holes for delivery of drugs at a high rate or pressure which the lumen could not sustain over its whole length. In some embodiments, the catheter may have two or more slits arranged proximally. The dimension of the slits and catheter material stiffness, diameters, and the thickness of the walls are selected such that they do not open inward under vacuum when blood is being sampled or under moderate pressures, such as when drugs are being infused slowly, but do open under pressures and flow rates that are needed for power injections and would cause a rupture of the catheter unless that flow were diverted or released into the vessel. If the properties are selected so that the catheter swells controllably under the designed pressure, this opens the slits even further and enables increased flow. The geometry and properties of the slits and the catheter determine the opening pressure, and the number of slits determines the flow rates that can be accommodated at a given pressure. This embodiment can be used with any existing or novel methods of insertion, sterile field establishment and other procedural aspects to limit the need for the majority of the catheter length to experience high pressures when injecting at high flow rates.

In some embodiments, the catheter **50, 60** may have additional coatings such as, but not limited to, antibacterial coatings, antifibrotic coatings, or other coatings that can facilitate sterility, ease of introduction into the blood vessel, or long-term use in the patient. In some embodiments, the catheter may be coated with a component or layer provided to stiffen the catheter **50, 60,** and in certain embodiments, at least one longitudinal bore of the catheter may include a guidewire or other stiffening means removeably inserted into the longitudinal bore. Such a guidewire or stiffening means may provide ease of handling of the catheter **50, 60** during introduction into the blood vessel, and in some embodiments, the guidewire or stiffening means may be removed after deployment of the catheter **50, 60.** In other embodiments, the catheter may not include a guidewire or stiffening means. For example, in embodiments in which catheter **60** includes a distal lateral slit **618** at a distal end **616,** fluid pressure within the catheter created while the lateral slit is closed may be prevented to reduce or eliminate undulating, flapping, or whipping of the catheter **60** during deployment of the catheter even for single lumen catheters. Similarly, in multi-lumen catheters, a first lumen **513***a* may be configured to accept and deploy fluid through a distal port, and a second lumen **513***b* may be sealed and configured to accept a fluid that is not delivered to the patient, or have a very small distal opening so that high pressure is developed at modest flow raters. In such embodiments, fluid pressure within the second lumen **513***b* may sufficiently stiffen the catheter **50** so as to reduce or eliminate the need for guidewires or other stiffening means.

The geometry and shape of the catheter body may be designed to withstand pressure by incorporating multiple design factors, for example, burst strength of the material and incorporation of reinforcing materials. To achieve the high flow rates for injecting contrast for imaging procedures utilizing pressure injections, the catheters need to be capable of withstanding higher pressures, e.g., 2068 kPa or 2240 kPa (300 psi or 325 psi), making these catheters stiffer than catheters that are used for the lower flow rates required for medication infusion and blood withdrawal, e.g., about 69 kPa to about 690 kPa (10 psi to about 100 psi). In some embodiments, a distal portion of the catheter may be composed of a first material at a proximal end of the catheter capable of withstanding pressures up to 350 psi, or about 2068 kPa (300 psi) to about 2240 kPa (325 psi), and a more flexible second material capable of withstanding pressures up to 690 kPa (100 psi), or from about 69 kPa (10 psi) to about 690 kPa (100 psi). In other embodiments, the catheter may be composed of a single material but may be designed to include a first portion at a proximal end of the catheter having a wall thickness that is greater than a distal portion of the catheter. The greater thickness may allow the proximal portion of the catheter to withstand higher pressures than the distal end of the catheter. Typically, the wall thickness may allow the proximal end to withstand high pressures than the distal end of the catheter.

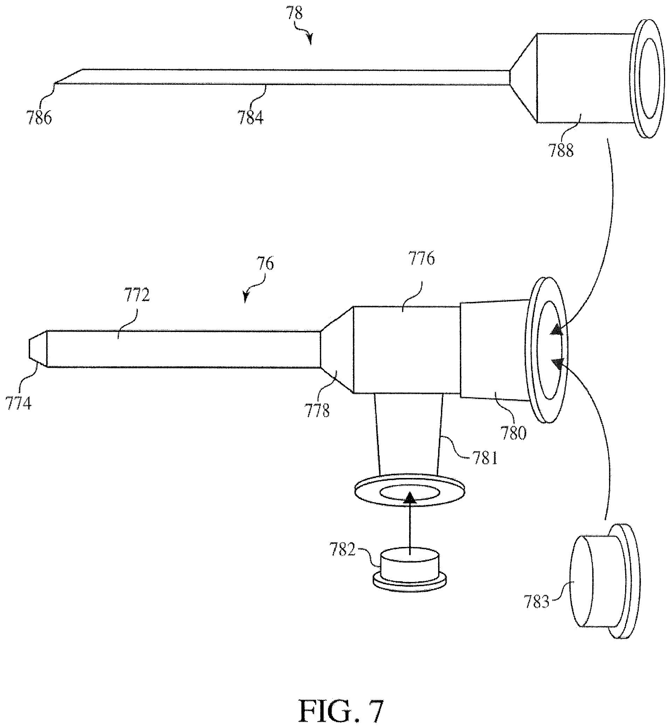

Further embodiments are directed to multi-port introducers and catheter retention devices designed to be used in conjunction with multi-port introducers. FIG. 7 shows an exemplary multi-port introducer **76** that includes an introducer tube **772** having a tapered distal end **774** designed to be introduced into a blood vessel. The proximal end of the introducer tube **772** may be operably connected to a multi-port fitting head **776,** which may include one or more fittings or ports for connecting with other apparatuses and devices. In FIG. 7, two fittings are provided **780** and **781.** The introducer tube **772** may be connected to the multi-port fitting head **776** by any means. For example, the multi-port fitting head **776** may be an integral part of the introducer tube **772** and, therefore, may be molded with the introducer tube **772.** In other embodiments, the multi-port fitting head **776** may be molded separately and fixedly attached to the introducer tube **772** using, for example, an adhesive, one or more connectors, or any other connector means known in the art or combinations thereof. In some embodiments, the introducer tube **772** and/or the multi-port fitting head **776,** or portions thereof, may include one or more Venturi features **778.**

The introducer tube **772** may have any inner diameter sufficiently sized to accommodate the catheter. For example, the introducer tube **772** may have an inner diameter sufficient to accommodate a 4, 5, 6, or 7 French catheters. In some embodiments, the introducer tube **772** may be an 18 gauge catheter having an inner diameter of about 1.3 mm (0.051 inches). In some embodiments, the introducer tube **772** may be sized such the lumen and opening in the distal end **774** of the introducer tube **772** provide sufficient space to allow fluid delivery through the introducer tube **772** during deployment of the catheter. Thus, fluid delivery can occur simultaneously with deployment of the catheter. In other embodiments, the distal end **774** of the introducer tube **772** may be sized to fit snuggly around the catheter during deployment, and simultaneous fluid deployment may be carried out through one or more holes or apertures in the introducer tube **772** that are positioned to allow fluid to flow from the introducer tube **772** into the blood vessel while the catheter is being deployed.

In some embodiments, the multi-port introducer **76** may include a Venturi feature **778** disposed between the multi-port fitting head **776** and the introducer tube **772** that is designed and arranged to interact with the catheter during deployment of the catheter. The Venturi feature **778** may generally have a substantially conical shape sized and configured to reduce the inner diameter of the multi-port fitting head **776.** In addition, the Venturi feature may be designed to increase the velocity of the fluid and thus the force pulling the catheter into the vein or reduce drag caused by the flow of fluid and friction created when the catheter contacts an inner surface of the Venturi feature **778** during insertion.

The multi-port fitting head **776** may include any number of ports or fittings, and as illustrated in FIG. 7, the exemplary multi-port fitting head **776** includes a first fitting **780** and a second fitting **781.** Such fittings may be any type of fittings known in the art and may be configured to removeably attach to any component known in the art. For example, in some embodiments, the fittings may be luer or screw type fittings, which may be configured to attach to, for example, commonly used IV tubes or syringes. In other embodiments, the fittings may be configured to attach directly to other medical devices such as, but not limited to, endoscopes. In still other embodiments, the fittings may be simple pressure fittings or other tube connectors.

The multi-port introducer **76** may be designed to be implanted into a patient in the same way as a typical angiocatheter such as the angiocatheter illustrated in FIG. 1. Therefore, the multi-port introducer **76** may include a number of peripheral parts that are present when the introducer is packaged but that can be removed after insertion into a blood vessel. For example, in certain embodiments, the multi-port introducer **76** may include a cap **782** designed to operably connect to and close off the second fitting **781,** and a cap **783** designed to operably connect to and close off the first fitting **780.** In particular embodiments, the multi-port introducer **76** may include a needle assembly **78** sized and configured to be received within a lumen of the multi-port introducer **76.** The needle assembly may generally include a needle **784** sized such that the distal pointed end **786** of the needle extends beyond the tapered distal end **774** of the introducer tube **772** when the needle **784** is received in the multi-port introducer **76.** The needle assembly **78** may further include a fitting or stopper **788** designed and configured to operably connect to at least a portion of the multi-port introducer **76** and hold the needle in place within the multi-port introducer **76.** In some embodiments, the fitting or stopper **788** may be held in place using simple pressure fittings, and in other embodiments, the fitting or stopper **788** may include a luer or screw-type fitting used to hold the needle assembly **78** on place during insertion into the blood vessel. In operation, the needle assembly **78** may provide sharp edges for facilitating entry into the blood vessel and through the skin. After the multi-port introducer **76** has been introduced into the blood vessel, the needle assembly may be removed leaving the multi-port introducer **76** in the blood vessel and providing an entry point into the blood vessel and circulatory system.

The first and second fittings **780, 781** may be initially covered using caps such as cap **782, 783** in **FIG. 7****,** and various common connectors may be used to connect the multi-port introducer to, for example, IV tubing or other medical devices. Therefore, a multi-port introducer may be used for introduction of fluids, medicaments, drugs, active agents, nutrients, and other fluids like a common angiocatheter.

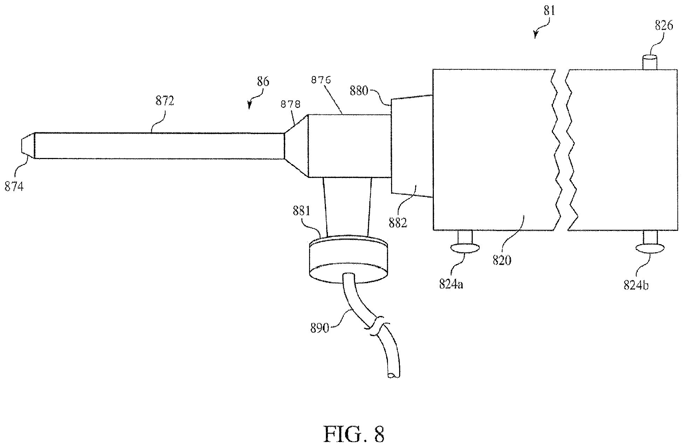

In particular embodiments, as illustrated in FIG. **8****,** the multi-port introducer **86** may be designed to link to a catheter retention device **81** such as those described herein. For example, in some embodiments, the second fitting **881** may be a luer or screw type fitting capable of attaching to a fluid source or tubing associated with a fluid source **890,** and the first fitting **880** may be configured to removeably attach to a catheter retention device **81.** The catheter retention device **81** may include any of the elements described herein and can associate with the multi-port introducer **86** through a distal fitting **882.** The catheter retention body **820** may house a catheter which is wound, coiled, folded, or otherwise retained in the catheter retention body **820,** such that a distal end of the catheter may enter the multi-port introducer **86** through the first fitting **880** and enter a blood vessel through the tapered distal end **874** of the introducer tube **872.** The catheter may be carried into and through the blood vessel by fluid introduced into multi-port introducer through the second fitting **881.**

Alternatively, the fitting **880** may be fitted with a "Y" fitting. A catheter may be inserted through one of the ports and the other can be used for delivering drugs as subsequently needed. In addition, the second Y-port could be used for insertion of another catheter with a similar or different length for injection of different fluids. Multiple Y-ports can be used as needed provided that the catheters are small enough to fit through the introducer. This has the benefit of being able to provide an additional lumen without having to make another IV stick in the patient or remove and existing catheter and introduce a new one.

In some embodiments, fluid flow from the second fitting **881** of the multi-port introducer **86** may be sufficient to carry the catheter into and through the blood vessel without introducing additional fluid through other ports. In other embodiments, the catheter retention device **81** may include one or more fittings capable of attaching to a fluid source and providing additional fluid that will flow through the catheter retention device 81 and multi-port introducer **86** to carry the catheter into the blood vessel. For example, in some embodiments, catheter retention device **81** may include one or more distal lateral fittings **824***a* and one or more proximal lateral fittings **824***b* through which fluid may enter the catheter retention body before flowing into the multi-port introducer **86** and into the blood vessel. The catheter retention device **81** may further include one or more purge vents **826** for releasing air trapped in the catheter retention body **820.**

The at least one portion of the multi-port introducer **86** may, generally, be sized to hold the catheter in place after deployment and may provide a seal around the outer circumference of the catheter to block flow of blood or other fluids from the introducer tube **872** into the catheter. For example, a seal may be provided at a junction between the introducer tube **872** and the Venturi feature **878** or the multi-port fitting head **876.** The proximal end of the catheter may remain in a portion of the first fitting **880** such that the proximal open end of the catheter can be accessed through the first fitting **880.** In some embodiments, the first fitting **880** may configured to hold the proximal end of the catheter in a space within the first fitting **880.** For example, in certain embodiments, the first fitting **880** may include a conical or spherical cavity at a proximal end of the fitting sized to receive and hold an end of the catheter. A clamp or other means for holding the catheter in the cavity may be provided at a distal end of the cavity to hold the proximal end of the catheter in place within the cavity while allowing the lumen of the catheter to remain free from obstruction. In some embodiments, the clamp may be a manual clamp. In operation, the catheter may be deployed from a catheter retention device through a multi-port introducer and into a blood vessel, and a clamp on the multi-port introducer may be engaged to fixedly hold the catheter in the multi-port injector. The catheter retention device may then be removed from the multi-port introducer with a length of catheter remaining in the catheter retention device, and the catheter may be cut near the multi-port introducer freeing the catheter retention device while leaving the catheter in place for the administration of medical fluids. Alternatively, the extra length of catheter may be coiled and attached to the patient's limb or body, or may be retained in the catheter retention device and the catheter retention device is attached securely and sterilely to the patient's limb or body.

In certain embodiments as illustrated in FIG. **7****,** the first fitting **780** may further include a cap **783** configured to seal the first fitting **780** and eliminate fluid flow through release of fluid from the multi-port introducer **76** after the catheter has been deployed. The cap **783** may be placed or replaced over the catheter after it has been deployed to protect the catheter from contamination when not in use. In such embodiments, the catheter may be deployed using the catheter retention device, and the catheter retention device can be removed while the catheter remains deployed in the blood vessel.

In particular embodiments, the multi-port introducer may be configured to be removed from the blood vessel following deployment of the catheter. For example, in some embodiments, the introducer tube of the multi-port introducer may be splittable such that following deployment of the catheter, the introducer tube may be removed by breaking the introducer tube along a longitudinal axis and removing the remaining halves from the insertion site. In such embodiments, the multi-port fitting head may remain associated with and/or attached to the catheter. In some embodiments, the catheter retention body may be attached to the multi-port fitting head, and in other embodiments, the catheter retention body may be removed before or after the introducer tube is removed.

In those embodiments where the introducer remains in the body, the distal end_in the vessel through which the more distally deployed catheter exits is an important aspect of this invention. If the introducer is a standard angiocath, the catheter is a relatively stiff material and the distal opening of the angiocatheter is of a relatively fixed diameter. This means that fluid can be injected around the catheter, and that there is the possibility for clot formation over time. Alternatively, the introducer or distal end of the introducer of this invention can be made of silicone or other relatively elastomeric material such that it is opened by the flow of fluid to transmit the catheter distally, and then closed to seal against the outer diameter of the catheter when flow is absent. By closing to reduce or eliminate the ingress of blood, the likelihood of clot formation in the introducer lumen is significantly reduced. To inject fluids through the introducer and into the patient, applying sufficient pressure will expand the introducer and conduct the fluid into the patient's vessel. Alternatively, slits or other openings may be provided in the wall of the introducer to transmit the injected fluid into the patient's vessels.

The multi-port introducers of various embodiments may include any number of additional features provided to facilitate ease of use or provide improved control of fluid flow through either the introducer tube or a catheter extending through the multi-port introducer. For example, in some embodiments, the introducer tube may include one or more discharge ports **990** that can be adjusted using a discharge button **991** located on a more distal portion of the multi-port introducer **96.** The discharge port **990** may, generally, be one or more lateral openings such as longitudinally adjustable slits or variable window apertures that are positioned on a portion of the introducer tube **972.** The discharge button **991** may twist or slide to be activated and may increase the flow of fluid into the blood vessel by allowing fluid from the multi-port introducer **96** to exit through the discharge ports **990** while simultaneously reducing the amount of fluid passing over the catheter thus slowing the deployment rate of the catheter.

In some embodiments, the multi-port fluid port may further include a compression brake and adjustable iris located between the first fitting and the introducer tube designed and configured to contact the catheter and slow the rate of deployment by way of friction as the catheter is deployed by depressing the brake or iris. The Venturi iris can be constructed to either compress inward or rotate around the body of the catheter. When activated, the opening of the Venturi iris widens and reduces the pressure drop between the inflow and out flow regions and reducing the force by the fluid flow applied to the catheter.

The various portions of the devices described above, including the angiocatheter or multi-port introducer and the catheter retention device may be combined to provide a single device, or in some embodiments, each element may be separate devices that are assembled during use. In other embodiments, the various components may be combined in a kit including one or more angiocatheters, one or more multi-port introducers, one or more catheter retention devices, fittings, tubing, and other components that may be useful for introducing a catheter into a blood vessel using the devices described above, and any combination of such components. As mentioned above, in various embodiments, the catheter may be pre-assembled, sterilized, and sealed in the catheter retention body of the catheter retention device. The catheter can remain inside a sterile container or package until it is inserted into the patient, so that the sterile field need not be much larger than that of a simple IV catheter (which typically involves just washing around the introduction site).

In some embodiments, the catheter may be processed before being packaged into the catheter retention body or the catheter may be packaged with additional components that may ease introduction of the catheter or provide other improvements. For example, in certain embodiments, a lubricant may be included in the sterile packaging to reduce any potential friction during deployment of the catheters. Similarly, a coating designed to reduce friction during deployment may be applied to the catheters during or prior to packaging.

Various embodiments are directed to examples for deploying a catheter. In general such examples include introducing a catheter into a blood vessel at an introduction site and simultaneously introducing a fluid into the blood vessel at the introduction site. In certain embodiments, simultaneous introducing of the catheter and fluid at a common introduction site can be carried out using a multi-port introducer such as those described above. In other embodiments, a multi-port introducer and a catheter retention device or a catheter retention device and a common angiocatheter can be used to simultaneously introduce a catheter and fluid into a blood vessel at a common introduction site. Without wishing to be bound by theory, the introduction of fluid with the catheter may allow the catheter to be carried through a blood vessel with the flow of blood through the blood vessels to the deployment site where it can be used for the deployment of drugs or other medical fluids without providing an external pushing force. The fluid flowing into the blood vessel may increase the blood volume and enlarge or distend the blood vessels to improve blood flow and assist in movement of the catheter through the blood vessels. Therefore, embodiments of the methods may be used for deployment of catheters through veins, which generally exhibit weaker blood flow, as well as arteries, and in certain embodiments, the methods may be directed specifically to introducing a catheter into a vein.

The flow rate and volume of fluid introduced during delivery of the catheter may vary among embodiments and may depend on various factors including, for example, restraining forces, the size of the catheter, the distance from the insertion site the catheter must travel for proper placement, the size of the vessels, amount of flex or expansion desired by the walls of the vein into which the catheter is being introduced, and the like and combinations thereof. In general, the flow rate should be sufficient to urge the catheter through the patient's vein and through any insertion apparatus such as an angiocatheter, a multi-port introducer, or either an angiocatheter or a multi-port introducer and a catheter retention device.

The flow rate may further off set restraining forces. As used herein, "restraining force" may be any force that slows or could slow movement of the catheter into the blood vessel. For example, restraining forces can be created by the friction as the catheter moves through an insertion apparatus such as an angiocatheter, a multi-port introducer, or either an angiocatheter or a multi-port introducer and a catheter retention device. Restriction forces further include manual restraint of the catheter caused by friction as the catheter is removed from a storage container or uncoiled from a spool. Limiting the play out of catheter, for example, a motion controlled or motion limited spool can also be considered a source of restraining force. Still other sources of restraining force include handling of the catheter by the clinician as it is introduced into the insertion apparatus. Further sources of restraining forces include for example, pinch clamps or friction wheels.

In some embodiments in which the catheter is deployed through an angiocatheter or a multi-port introducer, the angiocatheter or multi-port introducer may be removed from the insertion site after deployment. In other embodiments, the angiocatheter or multi-port introducer head may remain inserted into the blood vessel after deployment and may continue to be used for delivery of fluids into the blood vessel. For example, in some embodiments, methods may include the steps of introducing or deploying a catheter into a blood vessel through an angiocatheter or multi-port introducer and introducing fluid through the angiocatheter or multi-port introducer after deployment of the catheter. In some embodiments, the fluid introduced into the blood vessel after deployment may be saline, nutrient fluids such as glucose, or other medical fluid that can be continually introduced into a patient. In other embodiments, the angiocatheter or multi-port introducer may be used for locally delivering fluids such as, for example, contrasting agents for scans or other tests, or drugs or other active agents, or combinations of these with saline or other medical fluids. In certain embodiments, anti-fibrotic agents may be administered through the angiocatheter or multi-port introducer, and the anti-fibrotic agent may wash over the catheter mitigating the fibrotic response and reducing fibrous tissue deposits on the external surface of the catheter to reduce thrombosis or catheter occlusion. Therefore, the catheter may remain in place for a longer period of time than catheters that do not remain associated with an angiocatheter or multi-port introducer that allows for fluid to be introduced at an insertion site while a catheter is in place at the insertion site.

More particular embodiments include the steps of inserting an angiocatheter or multi-port introducer into a blood vessel, and in some embodiments, securing the angiocatheter or multi-port introducer using standard means such as taping to the patient's skin with medical tape. The method may include connecting the angiocatheter or multi-port introducer to a fluid source and providing a flow of fluid through the angiocatheter or multi-port introducer. Providing fluid flow may be accomplished by any means. For example, in some embodiments, fluid flow may be provided using saline from an IV bag and associated tubing that relies on gravity. In other embodiments, fluid flow may be provided by a pump that pushes or pumps fluid from a reservoir through the angiocatheter or multi-port introducer. In still other embodiments, flow of fluid may be provided using a syringe or other manual device. A catheter may then be inserted into the angiocatheter or multi-port introducer while simultaneous fluid flow is maintained through the angiocatheter or multi-port introducer. The catheter is carried by the flow of fluid through the angiocatheter or multi-port introducer and through the blood vessels by the fluid flow.

In some embodiments, the step of inserting a catheter into the angiocatheter or multi-port introducer can be facilitated with a catheter retention device such as those described above. In such embodiments, after inserting the angiocatheter or multi-port introducer into the blood vessel, the method may include attaching a catheter retaining device housing a catheter to the angiocatheter or multi-port introducer and initiating a flow of fluid through the angiocatheter or multi-port introducer, the catheter retaining body, or both. The flow of fluid may then carry the catheter into the blood vessel effecting deployment of the catheter.

In certain embodiments, the catheter retention device may include a number of fittings or purge vents. Referring to the catheter retention device of FIG. 3A, for example, such methods may include operably connecting the catheter retention body **31** to the angiocatheter or multi-port introducer, opening a purge vent **327** on a catheter retention body **320,** and filling the catheter retention body **320** with fluid. As fluid is introduced into the catheter retention body **320,** air from inside the catheter retention body **320** can be pushed out through the purge vent **327** thereby removing air from the catheter retention body **320.** When fluid begins flowing through the purge vent **327,** the purge vent **327** may be closed, and further fluid introduced into the catheter retention body **320** can flow into the angiocatheter or multi-port introducer carrying the catheter from the catheter retention body **320** into the angiocatheter or multi-port introducer and into the blood vessel. In some embodiments, deployment of the catheter may stop when the a bulge or stopper associated with a proximal end of the catheter reaches a position on the catheter retention device, angiocatheter, or multi-port introducer that cannot allow the bulge or stopper to pass.

After deployment of the catheter, the catheter retention device may be removed from the angiocatheter or multi-port introducer, and in some embodiments, the catheter may be secured to the angiocatheter or multi-port introducer using a cap. The catheter may be accessed as necessary by removing the cap or attaching a delivery device through the cap. As discussed above, fluids such as saline, active agents, drugs, or contrast agents can be introduced into the patient through the catheter while the angiocatheter or multi-port introducer remains in place, and saline, active agents, drugs, or contrast agents can be introduced into the blood vessel through the angiocatheter or multi-port introducer while the catheter remains in place. In various embodiments, the catheter may be sized to be positioned at a specific location within the body of the patient such as near a particular organ or injury, for example, near the patient's heart to effectuate localized delivery of the active agent, drug, or contrast agent introduced through the second catheter.

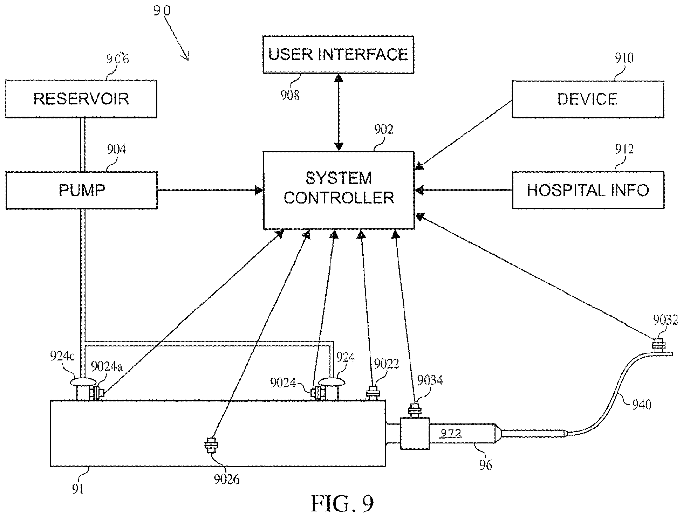

While the catheter and deployment system described herein can be operated manually using existing discrete ancillary devices and equipment such as angiocatheters, ultrasound imagers, and hand held syringes with saline, there can be a benefit in standardization of care and ease of use that comes with using an integrated electromechanical system. FIG. **9** shows a system that can be used to deploy the catheters of various embodiments. Such systems **90** may include, for example, a system controller **902,** electronically connected to one or more pumps **904** that can be fluidly connected to a fluid reservoir **906** or other source of injectable fluid. The system controller **902** may be configured to receive information about the catheter **940,** catheter retention body **91,** and insertion device, the patient, the procedure, and other parameters to determine the volumes and flow rates to be delivered over time. In addition, the system controller **902** may receive information from a user interface **908,** information tags on devices connected to the system controller such as RFID tags or bar codes **910,** Wi-Fi or other internet links to hospital information systems **912,** or combinations thereof.

In some embodiments, information about the catheter, catheter retention body, and insertion device, the patient, the procedure, and other parameters may be received by the control system **902.** For example, the control system **902** may be operably connected to the catheter retention body **91** through one or more catheter play-out monitors **9022** on, one or more flow port monitors **9024** associated with fluid ports **924,** pressure sensors **9026** on an inner surface of the catheter retention body **91,** and like and combinations thereof. In some embodiment, the system **90** may further include a tip location monitor **9032** or measurement system **9034** associated with the catheter **940.** A wide variety of tip location methods are currently used and can be incorporated into the systems of embodiments. In certain embodiments, the system **90** may use information received from a play-out monitor **9022** or measuring device **9034** and a tip location monitor **9032** to control the fluid flow by modulating the pump **904** or operating friction augmentation devices on the catheter retention body **91.** The information may be used by the operator and/or a computer program to optimize the tip location. In some embodiments, the tip location can be verified via a method such as a chest X-ray following deployment, and the tip location can be adjusted using the system if the deployment system **90** has not been removed. Alternatively, the catheter can be pulled back or moved forward if sterility has been maintained.

It will be appreciated that various of the above-disclosed and other features and functions, or alternatives thereof, may be desirably combined into many other different systems or applications. It will also be appreciated that various alternatives, modifications, variations or improvements therein may be subsequently made by those skilled in the art which are also intended to be encompassed by the disclosed embodiments. If not otherwise stated herein, it may be assumed that all components and/or processes described heretofore may, if appropriate, be considered to be interchangeable with similar components and/or processes disclosed elsewhere in the specification, unless an express indication is made to the contrary.

## Claims

1. A catheter retention device (21) comprising:
a catheter retention body (220) having a proximal end and a distal end, the catheter retention body (220) defining a lumen designed and configured to receive a catheter (240);
a fluid source that provides a fluid at a fluid flow rate which is greater than a flow of blood in a vein;
one or more proximal fittings operably connected to the catheter (240), and providing fluid communication from an exterior of the catheter retention body (220) to an interior of the catheter (240);
at least one distal lateral fitting (324) on a distal portion of the catheter retention body (220), the at least one distal lateral fitting (324) being designed and configured to couple to the fluid source and introduce the fluid into a distal portion of the lumen of the catheter retention body (220); and
a distal fitting (222) at the distal end of the catheter retention body (220), the distal fitting (222) defining a distal aperture through which the fluid and the catheter (240) exit the lumen of the catheter retention body (220), wherein the fluid flowing through the distal aperture provides substantially all of a force required to expel the catheter (240) from the catheter retention body (220) and provides a pulling force on the catheter (240) in a venous blood vessel related to the fluid flow rate and the flow of blood in the vein, wherein the fluid provides an augmented fluid flow rate in the venous blood vessel; and
at least one friction augmentation device positioned to contact the catheter (240) received within the lumen of the catheter retention body (220), wherein the at least one friction augmentation device controls a feed-out of the catheter (240) from the distal aperture of the catheter retention body (220) so the feed-out of the catheter is less than the augmented flow rate in the venous blood vessel.

2. The catheter retention device (21) as claimed in claim 1, further comprising the catheter (240) contained within the lumen of the catheter retention body (220).

3. The catheter retention device (21) as claimed in claim 2, wherein the catheter (240) is wound, coiled, or folded in the lumen of the catheter retention body (220).

4. The catheter retention device (21) as claimed in claim 2 or 3, wherein the catheter (240) is composed of a soft flexible material.

5. The catheter retention device (21) as claimed in any one of claims 2 to 4, wherein the catheter (240) does not include a guidewire.

6. The catheter retention device (21) as claimed in any preceding claim, further comprising one or more purge vents (327) disposed on the catheter retention body (220).

7. The catheter retention device (21) as claimed in any preceding claim, wherein the at least one distal lateral fitting (324) and the distal fitting (222) are selected from the group consisting of screw fittings, luer fittings, pressure fittings, snap fittings, and combinations thereof.

8. The catheter retention device (21) as claimed in any preceding claim, further comprising one or more valves selected from the group consisting of gate or slide valves, needle valves, check valves, diaphragm valves, butterfly valves, and combinations thereof positioned to regulate fluid flow into and through the catheter retention body (220).

9. The catheter retention device (21) as claimed in any preceding claim, further comprising one or more baffles, bulkheads, and combinations thereof disposed within the lumen of the catheter retention body (220).

10. The catheter retention device (21) as claimed in any preceding claim, further comprising texturing features on an inner surface of the catheter retention body (220), wherein the texturing features are selected from the group consisting of helical grooves, longitudinal grooves, circumferential grooves, rifling, projections, and combinations thereof.

11. The catheter retention device (21) as claimed in any preceding claim, further comprising:
a centering mandrel (321) sized to be received within the lumen of the catheter retention body (220); and
a mandrel clamp (323) positioned to hold the centering mandrel (321) at a center of a circumference of the catheter retention body (220).

12. The catheter retention device as claimed in claim 11, wherein the centering mandrel (321) is sized to be received within a lumen of the catheter (240).

13. The catheter retention device (21) as claimed in any preceding claim, wherein the at least one friction augmentation device is a pinch valve, an iris, a compression device, or a combination thereof.

## Patentansprüche

1. Katheterrückhaltevorrichtung (21), umfassend:
einen Katheterrückhaltekörper (220) mit einem proximalen Ende und einem distalen Ende, wobei der Katheterrückhaltekörper (220) ein Lumen definiert, das dafür ausgelegt und gestaltet ist, einen Katheter (240) aufzunehmen;
eine Fluidquelle, die ein Fluid mit einer Fluidflussrate liefert, die größer als ein Fluss von Blut in einer Vene ist;
ein oder mehrere proximale Anschlusselemente, die funktionsfähig mit dem Katheter (240) verbunden sind und Fluidverbindung von einem Außenraum des Katheterrückhaltekörpers (220) zu einem Innenraum des Katheters (240) bereitstellen;
wenigstens ein distales seitliches Anschlusselement (324) an einem distalen Teil des Katheterrückhaltekörpers (220), wobei das wenigstens eine distale seitliche Anschlusselement (324) zum Koppeln an die Fluidquelle und Einführen des Fluids in einen distalen Teil des Lumens des Katheterrückhaltekörpers (220) ausgelegt und gestaltet ist; und
ein distales Anschlusselement (222) an dem distalen Ende des Katheterrückhaltekörpers (220), wobei das distale Anschlusselement (222) eine distale Öffnung definiert, durch die das Fluid und der Katheter (240) aus dem Lumen des Katheterrückhaltekörpers (220) austreten, wobei das Fluid, das durch die distale Öffnung fließt, im Wesentlichen die gesamte Kraft liefert, die erforderlich ist, um den Katheter (240) aus dem Katheterrückhaltekörper (220) auszustoßen, und eine ziehende Kraft an dem Katheter (240) in einem venösen Blutgefäß liefert, die mit der Fluidflussrate und dem Blutfluss in der Vene verbunden ist, wobei das Fluid eine erhöhte Fluidflussrate in dem venösen Blutgefäß liefert; und
wenigstens eine Reibungserhöhungsvorrichtung, die für den Kontakt mit dem in dem Lumen des Katheterrückhaltekörpers (220) aufgenommenen Katheters (240) angeordnet ist, wobei die wenigstens eine Reibungserhöhungsvorrichtung die Ausgabe des Katheters (240) aus der distalen Öffnung des Katheterrückhaltekörpers (220) steuert, so dass die Ausgabe des Katheters kleiner als die erhöhte Flussrate in dem venösen Blutgefäß ist.

2. Katheterrückhaltevorrichtung (21) gemäß Anspruch 1, ferner umfassend den Katheter (240), der in dem Lumen des Katheterrückhaltekörpers (220) enthalten ist.

3. Katheterrückhaltevorrichtung (21) gemäß Anspruch 2, wobei der Katheter (240) in dem Lumen des Katheterrückhaltekörpers (220) gewickelt, gewendelt oder gefaltet ist.

4. Katheterrückhaltevorrichtung (21) gemäß Anspruch 2 oder 3, wobei der Katheter (240) aus einem weichen flexiblen Material besteht.

5. Katheterrückhaltevorrichtung (21) gemäß einem der Ansprüche 2 bis 4, wobei der Katheter (240) keinen Führungsdraht enthält.

6. Katheterrückhaltevorrichtung (21) gemäß einem der vorstehenden Ansprüche, ferner umfassend eine oder mehrere Spülöffnungen (327), die an dem Katheterrückhaltekörper (220) angeordnet sind.

7. Katheterrückhaltevorrichtung (21) gemäß einem der vorstehenden Ansprüche, wobei das wenigstens eine distale seitliche Anschlusselement (324) und das distale Anschlusselement (222) ausgewählt sind aus der Gruppe bestehend aus Schraub-Anschlusselementen, Luer-Anschlusselementen, Druck-Anschlusselementen, Schnapp-Anschlusselementen und Kombinationen davon.

8. Katheterrückhaltevorrichtung (21) gemäß einem der vorstehenden Ansprüche, ferner umfassend ein oder mehrere Ventile ausgewählt aus der Gruppe bestehend aus Schieber- oder Schiebeventilen, Nadelventilen, Rückschlagventilen, Membranventilen, Drosselventilen und Kombinationen davon, positioniert zum Regulieren von Fluidfluss in und durch den Katheterrückhaltekörper (220).

9. Katheterrückhaltevorrichtung (21) gemäß einem der vorstehenden Ansprüche, ferner umfassend eine oder mehrere Blenden, Trennwände und Kombinationen davon, die in dem Lumen des Katheterrückhaltekörpers (220) angeordnet sind.

10. Katheterrückhaltevorrichtung (21) gemäß einem der vorstehenden Ansprüche, ferner umfassend Texturierungselemente an einer Innenfläche des Katheterrückhaltekörpers (220), wobei die Texturierungselemente ausgewählt sind aus der Gruppe bestehend aus helikalen Rillen, Längsrillen, Umfangsrillen, Zügen, Vorsprüngen und Kombinationen davon.

11. Katheterrückhaltevorrichtung (21) gemäß einem der vorstehenden Ansprüche, ferner umfassend:
einen Zentrierdorn (321), der zur Aufnahme in das Lumen des Katheterrückhaltekörpers (220) bemessen ist; und
eine Dornklemme (323), die dafür angeordnet ist, den Zentrierdorn (321) bei der Mitte des Umfangs des Katheterrückhaltekörpers (220) zu halten.

12. Katheterrückhaltevorrichtung gemäß Anspruch 11, wobei der Zentrierdorn (321) zur Aufnahme in einem Lumen des Katheters (240) bemessen ist.

13. Katheterrückhaltevorrichtung (21) gemäß einem der vorstehenden Ansprüche, wobei die wenigstens eine Reibungserhöhungsvorrichtung ein Quetschventil, eine Iris, eine Kompressionsvorrichtung oder eine Kombination davon ist.

## Revendications

1. Dispositif (21) de rétention de cathéter, comprenant :
un corps (220) de rétention de cathéter comportant une extrémité proximale et une extrémité distale, le corps (220) de rétention de cathéter délimitant une lumière conçue et configurée pour recevoir un cathéter (240) ;
une source de fluide qui fournit un fluide à un débit de fluide qui est supérieur au débit de sang dans une veine ;
un ou plusieurs accessoires proximaux raccordés fonctionnellement au cathéter (240), et assurant une communication fluidique allant de l'extérieur du corps (220) de rétention de cathéter à l'intérieur du cathéter (240) ;
au moins un accessoire distal (324) latéral sur une partie distale du corps (220) de rétention de cathéter, ledit accessoire distal (324) latéral étant conçu et configuré pour s'accoupler à la source de fluide et introduire le fluide dans une partie distale de la lumière du corps (220) de rétention de cathéter ; et
un accessoire distal (222) à l'extrémité distale du corps (220) de rétention de cathéter, l'accessoire distal (222) délimitant une ouverture distale par laquelle le fluide et le cathéter (240) sortent de la lumière du corps (220) de rétention de cathéter,
dans lequel le fluide s'écoulant par l'ouverture distale fournit sensiblement la totalité de la force requise pour expulser le cathéter (240) du corps (220) de rétention de cathéter et fournit une force de traction sur le cathéter (240) dans un vaisseau sanguin veineux liée au débit de fluide et au débit de sang dans la veine,
dans lequel le fluide fournit un débit de fluide accru dans le vaisseau sanguin veineux ; et
au moins un dispositif d'augmentation de frottement placé pour être en contact avec le cathéter (240) reçu à l'intérieur de la lumière du corps (220) de rétention de cathéter,
dans lequel ledit dispositif d'augmentation de frottement commande la sortie du cathéter (240) de l'ouverture distale du corps (220) de rétention de cathéter pour que la sortie du cathéter soit inférieure au débit augmenté dans le vaisseau sanguin veineux.

2. Dispositif (21) de rétention de cathéter selon la revendication 1, comprenant en outre le cathéter (240) contenu à l'intérieur de la lumière du corps (220) de rétention de cathéter.

3. Dispositif (21) de rétention de cathéter selon la revendication 2, dans lequel le cathéter (240) est bobiné, lové ou plié dans la lumière du corps (220) de rétention de cathéter.

4. Dispositif (21) de rétention de cathéter selon la revendication 2 ou 3, dans lequel le cathéter (240) est composé d'un matériau flexible et doux.

5. Dispositif (21) de rétention de cathéter selon l'une quelconque des revendications 2 à 4, dans lequel le cathéter (240) ne comprend pas de fil guide.

6. Dispositif (21) de rétention de cathéter selon l'une quelconque des revendications précédentes, comprenant en outre un ou plusieurs évents (327) de purge disposés sur le corps (220) de rétention de cathéter.

7. Dispositif (21) de rétention de cathéter selon l'une quelconque des revendications précédentes, dans lequel ledit accessoire distal (324) latéral et l'accessoire distal (222) sont choisis dans le groupe constitué par des accessoires à vis, des accessoires Luer, des accessoires à pression, des accessoires à mise en place rapide et des combinaisons de ceux-ci.

8. Dispositif (21) de rétention de cathéter selon l'une quelconque des revendications précédentes, comprenant en outre un ou plusieurs robinets choisis dans le groupe constitué par des robinets-vannes à guillotine ou à lunette, des robinets à pointeau, des clapets anti-retours, des soupapes à diaphragme, des robinets à papillon et des combinaisons de ceux-ci, placés pour réguler le débit du fluide entrant dans le corps (220) de rétention de cathéter et le traversant.

9. Dispositif (21) de rétention de cathéter selon l'une quelconque des revendications précédentes, comprenant en outre une ou plusieurs chicanes, cloisons et combinaisons de celles-ci disposées à l'intérieur de la lumière du corps (220) de rétention de cathéter.

10. Dispositif (21) de rétention de cathéter selon l'une quelconque des revendications précédentes, comprenant en outre des éléments de texturation sur la surface intérieure du corps (220) de rétention de cathéter,
dans lequel les éléments de texturation sont choisis dans le groupe constitué par des rainures hélicoïdales, des rainures longitudinales, des rainures circonférentielles, des rayures, des saillies et des combinaisons de celles-ci.

11. Dispositif (21) de rétention de cathéter selon l'une quelconque des revendications précédentes, comprenant en outre :
un mandrin (321) de centrage dimensionné pour être reçu à l'intérieur de la lumière du corps (220) de rétention de cathéter ; et
un mors de serrage (323) placé pour maintenir le mandrin (321) de centrage au centre de la circonférence du corps (220) de rétention de cathéter.

12. Dispositif de rétention de cathéter selon la revendication 11, dans lequel le mandrin (321) de centrage est dimensionné pour être reçu à l'intérieur de la lumière du cathéter (240).

13. Dispositif (21) de rétention de cathéter selon l'une quelconque des revendications précédentes, dans lequel ledit dispositif d'augmentation de frottement est un robinet à manchon déformable, un iris, un dispositif de compression ou une combinaison de ceux-ci.
